# EUROPEAN PATENT APPLICATION

(11) **EP 2 039 775 A2**
(43) Date of publication of application: **25.03.2009**
(21) Application number: 08014934.7
(22) Date of filing: 22.08.2008
(51) Int. Cl.: C12P 5/02, C02F 11/04, C12M 1/113, C12N 1/20

(54) **Apparatus and method for manufacturing methane**

(30) Priority: 23.08.2007 JP 2007217301
(71) Applicant: Hitachi Engineering & Services Co., Ltd., Hitachi-shi, Ibaraki 317-0073 (JP); Hiroshima University, Higashi-Hiroshima-shi Hiroshima 739-8511 (JP)
(72) Inventor: Iwai, Kazumi Hitachi Engineering & Services Co.,Ltd., Hitachi-shi, Ibaraki 317-0073 (JP); Tanaka, Tadashi Hitachi Engineering & Services Co.,Ltd., Hitachi-shi, Ibaraki 317-0073 (JP); Satou, Tsutomu Hitachi Engineering & Services Co.,Ltd., Hitachi-shi, Ibaraki 317-0073 (JP); Takamura, Tetsuo Hitachi Engineering & Services Co.,Ltd., Hitachi-shi, Ibaraki 317-0073 (JP); Tayama, Osamu Hitachi Engineering & Services Co.,Ltd., Hitachi-shi, Ibaraki 317-0073 (JP); Nishio, Naomichi, Higashihiroshima-shi 739-0002 (JP)
(74) Representative: Beetz & Partner

(57) **Abstract**

An object of this invention is to provide an apparatus and a method for controlling the concentration of ammonia contained in a fermentation material processed in an ammonia fermenter to a prescribed value at a low cost. A means for attaining the object is to charge a fermentation material mixed with, as representative examples, the both or either of the bacteria named *Tepidimicrobium* sp. strain HUT8118 and deposited under the reference number NITE AP-390 at Patent Microorganisms Depositary in Incorporated Administrative Agency National Institute of Technology and Evaluation and the bacteria named *Tepidimicrobium* sp. strain HUT8119 and deposited under the reference number NITE AP-391 at Patent Microorganisms Depositary in Incorporated Administrative Agency National Institute of Technology and Evaluation into an ammonia fermenter and to keep the fermentation material at a prescribed temperature.

## Description

### FIELD OF THE INVENTION:

The present invention relates to an ammonia fermenter that utilizes reaction by microorganisms, an apparatus for manufacturing a flammable gas and a method for operating the same.

### RELATED ART:

In recent years, the productions of biogases including methane gas by fermenting sewage sludge, livestock excreta, food wastes, plants, or other organic substances and the utilization of the biogas energy have been increasing.

A gas generated from a fermentation material such as organic wastes does not always activate microorganisms and for example ammonia is predisposed to kill so-called methane bacteria that produce a methane gas.

Meanwhile, an amount of generated ammonia depends on an amount of a nitrogen component existing in a fermentation material. In other words, the nitrogen component existing in a fermentation material is decomposed by a function of microorganisms and transforms into ammonium ions that are ammonia nitrogen. The ammonium ions transform into nitrite ions or nitrate ions by the function of microorganisms such as ammonia oxidizing bacteria and the ions transform into nitric oxide, nitrous oxide, and finally nitrogen gas and are discharged. The ammonia generated in the process adversely affects microorganisms that conduct a methane fermentation.

Patent Document 1 (Japanese Patent Laid-open No. 2006-205017) discloses a method for processing organic wastes by anaerobic fermentation, which is characterized by including a first fermentation process for solubilizing the organic wastes and producing ammonia and hydrogen in order to prevent ammonia or the like from hindering a methane fermentation, a process for removing the produced hydrogen and ammonia, and a second fermentation process for subjecting the solubilized organic wastes from which the ammonia and the hydrogen are removed to the methane fermentation. Patent Document 1 describes that, in the process of removing ammonia from a fermentation material, an alkaline material such as calcium hydroxide is added in large quantities and heated and thereby ammonia is discharged as a gas. In the method therefore, an alkaline material has to be added continuously.

Patent Document 2 (Japanese Patent Laid-open No.2006-150159) discloses a methane fermentation processing system that has an energy converter to ferment organic wastes, to produce methane gas and to obtain thermal energy and electric energy from the methane gas and that utilizes the energy obtained with the energy converter.

In addition, non-Patent Document 1 (Biotechnology Vol. 85, No. 4, pp.171-173 (2007)) discloses a dry type methane fermentation process for dehydrated sludge, the process including an ammonia fermenter, a deammoniation tank, and a dry type methane fermenter.
[Patent Document 1] Japanese Patent Laid-open No.2006-205017
[Patent Document 2] Japanese Patent Laid-open No.2006-150159
[Non-Patent Document 1] Biotechnology Vol. 85, No. 4, pp.171-173 (2007)

### SUMMARY OF THE INVENTION:

As described in Patent Document 1 and non-Patent Document 1, it is effective to apply two-step processing comprising an ammonia fermentation and a methane fermentation to fermentation processing of organic wastes, and to apply deammoniation processing after the ammonia fermentation in order to prevent ammonia or the like from hindering the methane fermentation.

However, The deammoniation processing described in Patent Document 1 has difficulty in practical application because of a labor and a running cost required for the addition of an alkaline material. Further, the method described in non-Patent Document 1 is required to additionally install a deammoniation tank and an increase of an operation cost is a concern.

An object of the present invention is to provide at a low cost an apparatus and a method for controlling a concentration of ammonia contained in a fermentation material processed in an ammonia fermenter in the ammonia fermenter of an apparatus to produce a flammable gas through two-step fermentation processing comprising the ammonia fermentation and a flammable gas fermentation (a methane fermentation or the like) when a fermentation material such as organic wastes is fermented.
The present invention is characterized by being prepared so as to contain at least one kind of bacteria selected from among *Tepidimicrobium ferriphilum, Clostridium ultunense,* Peptostreptococcaceae bacterium 19gly, *Clostridium* sp. PO, *Sporanaerobacter acetigenes* Lup33, and *Acetanaerobacter* sp. in addition to one or both the bacteria named *Tepidimicrobium* sp. strain HUT8118 deposited under a reference number NITE AP-390 and *Tepidimicrobium* sp. strain HUT8119 deposited under a reference number NITE AP-391 at Patent Microorganisms Depositary (NPMD) in Incorporated Administrative Agency National Institute of Technology and Evaluation (NITE); to mix the one or more kinds of the bacteria into the fermentation material; to charge the fermentation material into a fermenter; to control the temperature of the fermentation material; to produce ammonia gas and to discharge the ammonia gas produced in the fermenter outside the fermenter through an exhaust port.

According to the present invention, it is possible to apply the ammonia fermentation by using the specific bacteria without solubilizing the fermentation material and to discharge the ammonia generated in the ammonia fermenter as ammonia gas outside the ammonia fermenter. Consequently, it is possible to remove ammonia generated in an ammonia fermenter at a low cost without requiring the addition of the alkaline material and the deammoniation tank. As a result, it is possible to prevent ammonia from hindering methane bacteria, to stabilize the methane fermentation and to produce methane gas efficiently, when the flammable gas such as methane gas is obtained by fermenting the fermentation material in the flammable gas fermenter at the second step.

### BRIEF DESCRIPTION OF THE DRAWINGS:

Fig. 1 is a general configuration view showing an ammonia fermenter according to a first embodiment of the present invention.
Fig. 2 is a general configuration view showing a flammable gas production system according to a second embodiment of the present invention.
Fig. 3 is a general configuration view showing an energy converter used in a flammable gas production system according to a third embodiment of the present invention.
Fig. 4 is a dendrogram of bacteria according to the present invention.
Fig. 5-1 is a graph showing measurement results of pHs in an ammonia fermenter according to the present invention.
Fig. 5-2 is a graph showing measurement results of total volumes of a gas generated in the ammonia fermenter according to the present invention.
Fig. 5-3 is a graph showing measurement results of volumes of carbon dioxide generated in the ammonia fermenter according to the present invention.
Fig. 5-4 is a graph showing measurement results of volumes of hydrogen generated in the ammonia fermenter according to the present invention.
Figs. 6A to 6C are graphs showing results of ammonia generation tests in a fermentation of poultry manure.

### DETAILED DESCRIPTION OF THE PREFERRED EMBODIMENTS:

The present invention is designed so as to use specific bacteria that can process the ammonia fermentation efficiently and to set conditions that can allow the bacteria to function most effectively.

Then, in the present invention, heat of reaction of the fermentation material itself is not used for the heat-retention of the fermentation material to the utmost in an ammonia fermenter and the fermentation material is maintained at a prescribed temperature by a temperature control means that uses an external heat.

Further, in the present invention, no chemicals are added in the ammonia fermenter. The preferable bacteria found here are important constituent elements of the present invention.

Representative examples of organic substances called biomass are livestock excreta, food residues, agricultural products, plants, and others. Such organic substances are hydrolyzed under prescribed conditions and acid such as butyric acid is produced. When methane bacteria acts on the acid, methane gas is produced.

However, the process requires a relatively long time and ammonia harmful to the methane fermentation is generated from the nitrogen component contained in the biomass. The ammonia has the function of killing the methane bacteria in the fermenter and prevents methane gas from being produced.

To cope with the problem, it has heretofore been attempted to reduce the amount of the nitrogen component contained in organic substances charged into the fermenter before the organic substances are charged into the methane fermenter in order to reduce the amount of ammonia generated in the flammable gas fermenter (here represented by the methane fermenter). Currently employed main means are a means for charging the fermentation material into the fermenter by mixing fermentation materials not containing nitrogen components and thus reducing the apparent concentration of the nitrogen component, or a means for reducing the apparent concentration of the nitrogen component with a large amount of dilution water.

However, any of those means causes the charged amount of the fermentation material to be processed to reduce and a large drawback thereof is that a desired amount of the flammable gas is not produced. Further, the adjustment of the nitrogen concentration with dilution water causes the amount of the digestive juice in the residues discharged from the flammable gas fermenter to increase and thus the problems are that the amount of the processed digestive juice increases and both the process cost and the operation cost increase considerably.

A dry type methane fermentation process does not use dilution water in order to adjust an ammonia concentration in the fermenter and is different from a wet type methane fermentation process that uses dilution water.

Consequently, in a system having only a dry type methane fermenter, technological measure to prevent ammonia generated from the fermentation material from hindering methane bacteria is required. In the cases of food residues and livestock excreta including a large amount of the nitrogen component in particular, to solve the problem is urgently needed. In order to solve the problem, it is effective to employ a two-step fermentation process including the ammonia fermentation and the methane fermentation, and to apply deammoniation processing after the ammonia fermentation as described in Patent Document 1 and non-Patent Document 1. In order to make the two-step fermentation process more practically applicable, it is important to operate the process at a low cost as described above.

In a preferable embodiment according to the present invention, a denitrification dry type methane two-step fermentation system is employed, wherein an anaerobic ammonia fermenter is installed on the upstream side of an anaerobic dry type methane fermenter, the nitrogen component included in a fermentation material is removed in the state of ammonia in the ammonia fermenter, that is, denitrification is applied, and the amount of the ammonia generated afterward in the dry type methane fermenter is reduced. The system enables it possible to optimize pH in the dry type methane fermenter and to prevent ammonia from hindering methane bacteria at a low cost.

In a preferable embodiment according to the present invention, the flammable gas production apparatus is configured by connecting the ammonia fermenter to the flammable gas fermenter in order to ferment sewage sludge, livestock excreta, food wastes, plants, or other organic substances efficiently and to stabilize the amount of the produced flammable gas.

The ammonia fermenter is installed on the upstream side from the flammable gas fermenter and anaerobic fermentation is carried out. In the ammonia fermenter, the nitrogen component included in organic substances is included in the form of protein or uric acid in many cases and the protein and the uric acid are predisposed to be hydrolyzed by an enzyme and to be formed into ammonia. It is known that ammonia hinders methane bacteria necessary for the methane fermentation and thus methane gas is produced more stably as the concentration of ammonia in the fermentation material reduces.

There are data used as an indication showing the degree of influence of an ammonia concentration in the fermentation material used for the methane fermentation on the amount of the produced methane. According to the data, the concentration of ammonia in the fermentation material suitable for the methane fermentation is 3500 mg/kg or less.

Then in a preferable embodiment according to the present invention, the ammonia component in the fermentation material is removed as an ammonia state and the retention time in the ammonia fermenter is adjusted so that the ammonia concentration may be 3500 mg/kg or less, namely 0.35% or less, in the succeeding flammable gas fermenter. Here, in the ammonia fermenter, the retention time is adjusted and the fermentation material is deammoniated. It is desirable however to adjust the rate and the amount of the deammoniated fermentation material charged into the flammable gas fermenter so that the fermentation material may be fermented into methane under desirable conditions even in the flammable gas fermenter as it will be described later.

Further, in the present invention, bacteria suitable for the ammonia fermentation are specified. As a result, two kinds of bacteria are found to be suitable. Then the two kinds of bacteria are named *Tepidimicrobium* sp. strain HUT8118 and *Tepidimicrobium* sp. strain HUT8119, respectively. Then the two kinds of bacteria are deposited at Patent Microorganisms Depositary in Incorporated Administrative Agency National Institute of Technology and Evaluation and obtain the reference number NITE AP-390 and the reference number NITE AP-391, respectively. Those are bacteria derived from poultry manure and digestive sludge. The accession numbers are NITE P-390 and NITE P-391 at NPMD.

Bacteria suitable for the ammonia fermentation according to the present invention are not limited to the above two kinds but *Tepidimicrobium, ferriphilum* related to the above two kinds of bacteria, *Clostridium ultunense,* Peptostreptococcaceae bacterium 19gly, *Clostridium* sp. PO, *Sporanaerobacter acetigenes* Lup33, and *Acetanaerobacter* sp. are also effective. Those bacteria live also in poultry manure and digestive sludge in many cases.

### [First Embodiment]

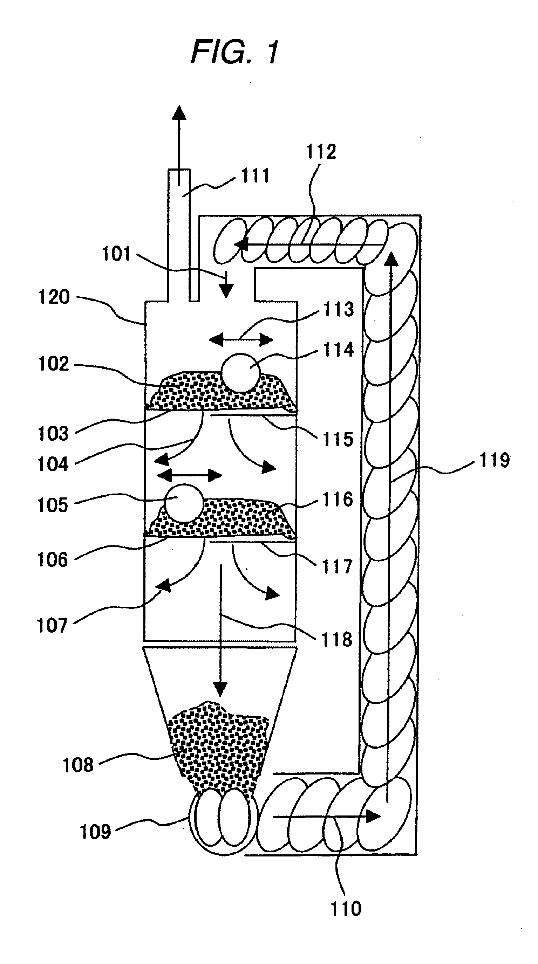

A schematic configuration view of an ammonia fermenter according to the present invention is shown in Fig. 1.

The interior of an ammonia fermenter main body 120 is divided into plural stages in the vertical direction. In the embodiment shown in Fig. 1, the interior is divided into two stages. Then, the ammonia fermenter is provided with an agitating and conveying means 109 for mixing fermentation materials 102 and 116 at the lower part of the ammonia fermenter main body 120 and for conveying the fermentation materials 102 and 116 to the exterior of the ammonia fermenter main body 120, and conveying means 110, 119, and 112 for conveying the agitated fermentation material 108 to the upper stage of the ammonia fermenter main body 120. The first stage of the ammonia fermenter main body 120 is formed with floor faces 103 and 115 and the second stage thereof is formed with floor faces 106 and 117. Further, at the stages, rollers 114 and 105 to evenly pile up the contained fermentation materials 102 and 116 are installed, respectively. Furthermore, an exhaust port 111 to discharge generated ammonia gas outside the ammonia fermenter is installed at the upper part of the ammonia fermenter main body 120.

The fermentation materials 102 and 116 are retained at the stages respectively as shown in Fig. 1. After the fermentation materials 102 and 116 are fermented for a certain period of time, the fermentation materials 102 and 116 at the respective stages are dropped to and gathered at the lower part of the ammonia fermenter main body 120 as shown with the arrow 118 and agitated and mixed by the agitating and conveying means 109. The agitated and mixed fermentation material 108 is lifted up to an inlet 101 of the ammonia fermenter main body 120 again through the conveying means 110, 119, and 112. The fermentation material lifted up to the inlet 101 is distributed to the stages again.

When the fermentation further advances, the fermentation materials 102 and 116 are gathered at the lower part of the ammonia fermenter main body 120 again, mixed and conveyed, and lifted up to the uppermost part of the ammonia fermenter main body 120 again. By repeating such processes, the nitrogen components in the fermentation materials 102, 116, and 108 are evenly transformed into ammonia and discharged from the exhaust port 111 outside the ammonia fermenter.

The fermentation materials 102, 116, and 108 the nitrogen components of which are reduced in the ammonia fermenter are conveyed to a methane fermenter of the next fermentation step. Here, when the fermentation materials 102 and 116 at the respective stages are gathered at the lower part of the ammonia fermenter main body 120, the floor faces 103, 115, 106, and 117 at the respective stages open downward as shown with the reference numerals 104 and 107 and the fermentation materials 102 and 116 fall free. It goes without saying that the mixing is carried out also during the free-fall process. The fermentation material 108 is lifted up to the uppermost part of the ammonia fermenter main body 120 again and charged into the ammonia fermenter main body 120 through the inlet 101. Here, the fermentation materials 102 and 116 may be flattened with the rollers 114 and 105 in order to make the thickness uniform.

Here, a means for free-falling a fermentation material is called a free-falling means and the agitating and conveying means 109 and the conveying means 110, 119, and 112 are called a mechanical agitating means. Those means constitute a means for mixing fermentation materials.

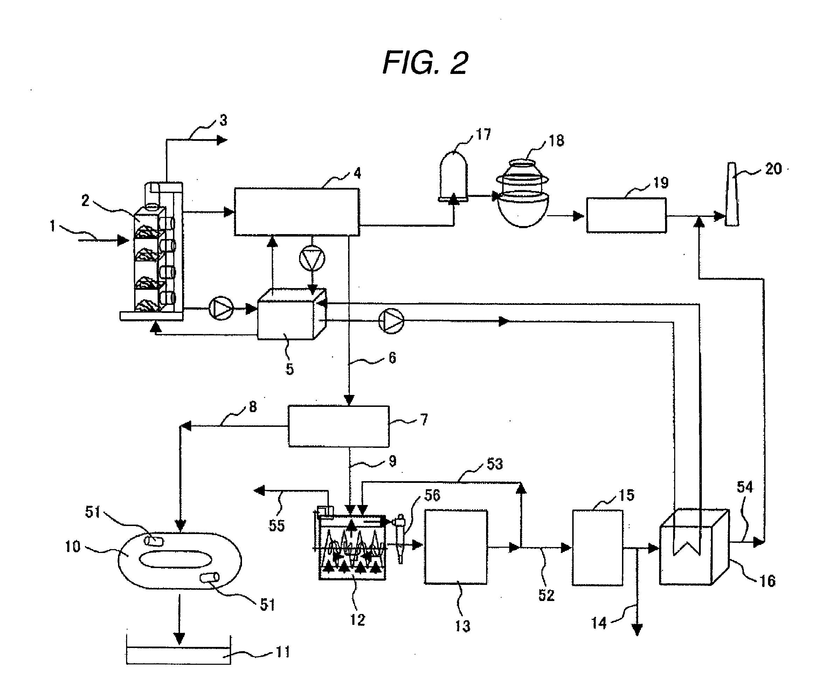

Note that, although they are not shown in Fig. 1, an inlet for a fermentation material, a means for conveying the fermentation material to a methane fermenter, a means for controlling (adjusting) the temperature of the ammonia fermenter, a temperature measuring means, an ammonia concentration measuring means, and a pH measuring means are installed in the ammonia fermenter. The means for conveying the fermentation material to a methane fermenter is branched from the conveying means 119 and conveys the deammoniated fermentation material to the methane fermenter as shown in Fig. 2.

Generally in aerobic fermentation, organic matters in a fermentation material also react simultaneously with the ammonia fermentation. The reaction of organic matters causes the volume of flammable gas produced in the methane fermentation that is carried out after the ammonia fermentation to reduce, and thus does not conform to the purpose, namely the production of the flammable gas by the methane fermentation.

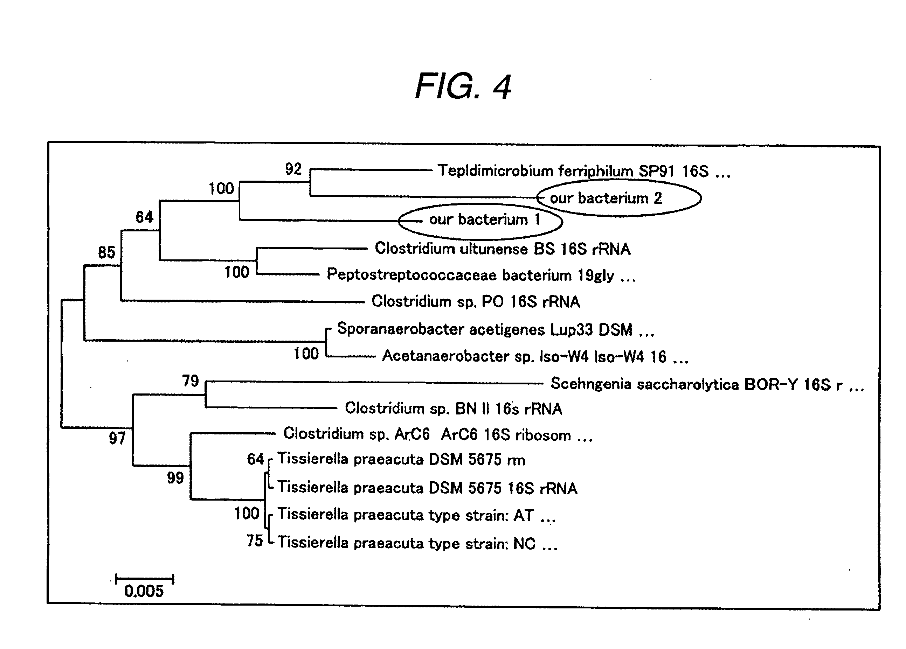

In view of the above situation, in the present invention, the anaerobic fermentation is employed, microorganisms suitable for the anaerobic ammonia fermentation are surveyed in order to mostly carry out the only ammonia fermentation, and bacteria shown in the dendrogram of Fig. 4 are specified.

That is, the specified bacteria are two kinds of bacteria suitable for the ammonia fermentation having a homology of 93% at a cultivation temperature of 65°C and a homology of 92% at a cultivation temperature of 55°C in comparison with 16S rDNA base sequence of *Tepidimicrobium ferriphilum* sp.91. The former bacterium is hereunder referred to as our bacterium 2 (*Tepidimicrobium* sp. strain HUT8118) and the latter bacterium is referred to as our bacterium 1 (*Tepidimicrobium* sp. strain HUT8119). Then the two kinds of bacteria are deposited at Patent Microorganisms Depositary in Incorporated Administrative Agency National Institute of Technology and Evaluation, and obtain the reference number NITE AP-390 and the reference number NITE AP-391, respectively. The date of the reception of the two kinds by Patent Microorganisms Depositary is August 3, 2007.

In addition, the ammonia fermenter 120 may contain, for example, *Tepidimicrobium ferriphilum, Clostridium ultunense,* Peptostreptococcaceae bacterium 19gly, *Clostridium* sp. PO, *Sporanaerobacter acetigenes* Lup33, and Acetanaerobacter sp. and others.

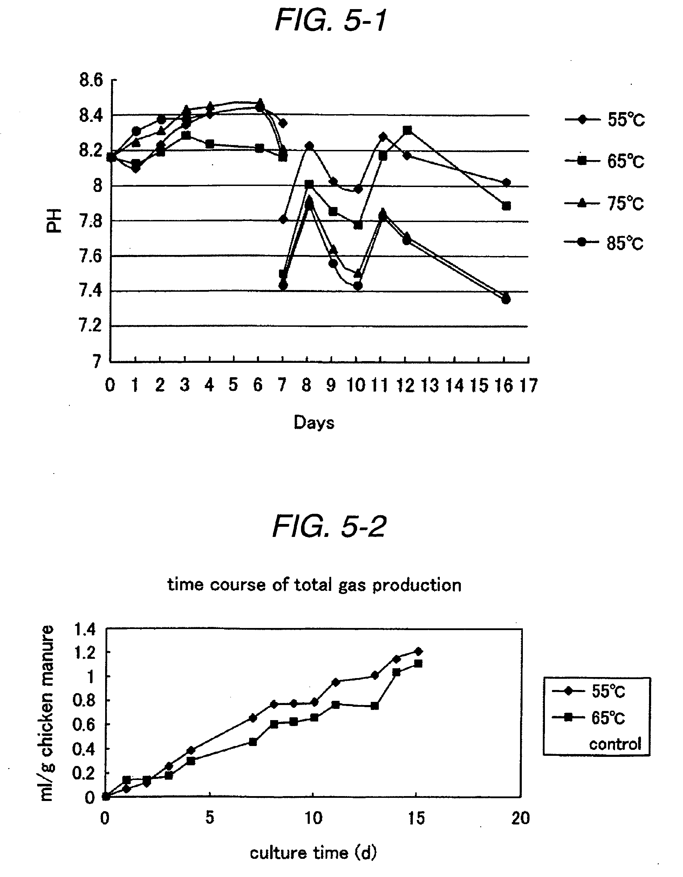

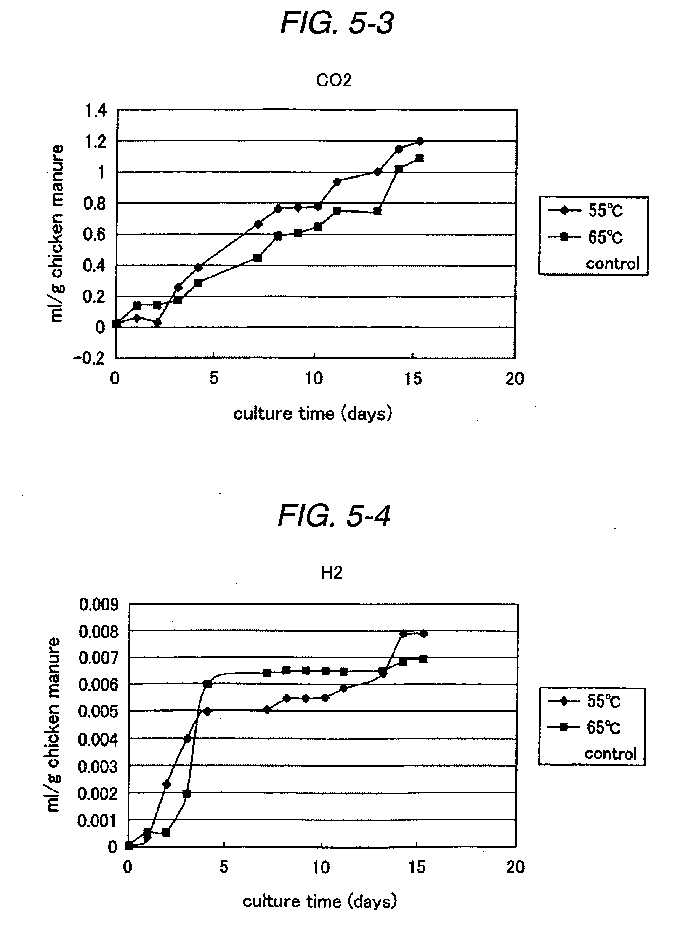

The results of ammonia fermentation tests with our bacterium 1 (fermentation temperature 55°C) and our bacterium 2 (fermentation temperature 65°C) are shown in Figs. 5-1, 5-2, 5-3, and 5-4.

Fig. 5-1 shows the variation of pH in an ammonia fermenter at various fermentation temperatures. The horizontal axis shows the number of days and the vertical axis shows a pH (a hydrogen-ion concentration).

The pH suitable for the ammonia fermentation in a fermenter is in the alkaline region and desirably in the range of pH 8 to pH 8.5. When the results are examined from this point of view, pH is nearly in the desirable range when the fermentation temperature is controlled to 55°C and 65°C but pH lowers and approaches neutrality after seventh day when the fermentation temperature is raised to 75°C or higher.

Here, the fermentation temperature at which the pH is nearly in the range of pH 8 to pH 8.5 may vary in accordance with the kind (the difference in the content of organic nitrogen and the amount of water) and the charged amount of a fermentation material and the like. Consequently, it is desirable to confirm beforehand the fermentation temperature at which pH is nearly in the range of pH 8 to pH 8.5 by a test or the like. According to our test results, it is known that the range of pH 8 to pH 8.5 can be maintained at least at a temperature close to 55°C or 65°C as shown in Fig. 5-1.

Further, when the retention time is within 6 days, pH can be maintained nearly in the range of pH 8 to pH 8.5 even at the fermentation temperature of 75°C or 85°C as shown in Fig. 5-1, but after the seventh day, pH approaches the neutral region particularly at the fermentation temperature of 75°C or 85°C, and hence a desirable retention time of the fermentation material in the ammonia fermenter is 6 days.

The present invention is very advantageous from the viewpoints of labor and a running cost because a deammoniation tank is not used basically and continuous charge of chemicals such as an alkaline material is not required. When the pH in the ammonia fermenter suddenly lowers to less than 7.5 however, it is effective to raise the pH to an appropriate level by charging an alkaline material into the ammonia fermenter.

Fig. 5-2 shows the results of the fermentation tests when poultry manure is used as the fermentation material and bacteria according to the present invention are used. The horizontal axis shows the number of days and the vertical axis shows a total produced gas volume per one gram of poultry manure.

It is understood that the produced gas volume increases as the fermentation time increases at the fermentation temperature of 55°C or 65°C. It has been found that gas is scarcely produced at the fermentation temperature of 75°C or 85°C although it is not shown in the figure.

Figs. 5-3 and 5-4 show the variations of the volumes of the produced CO₂ and H₂ in the fermentation tests respectively when poultry manure is used as the fermentation material and bacteria according to the present invention are used. In each of the figures, the horizontal axis shows the number of days and the vertical axis shows a produced gas volume per one gram of poultry manure.

In the figure, it has been confirmed that gas is generated markedly at the fermentation temperature of 55°C or 65°C. That is, it has been found that preferable fermentation temperatures includes 55°C and 65°C and a temperature lower than 75°C.

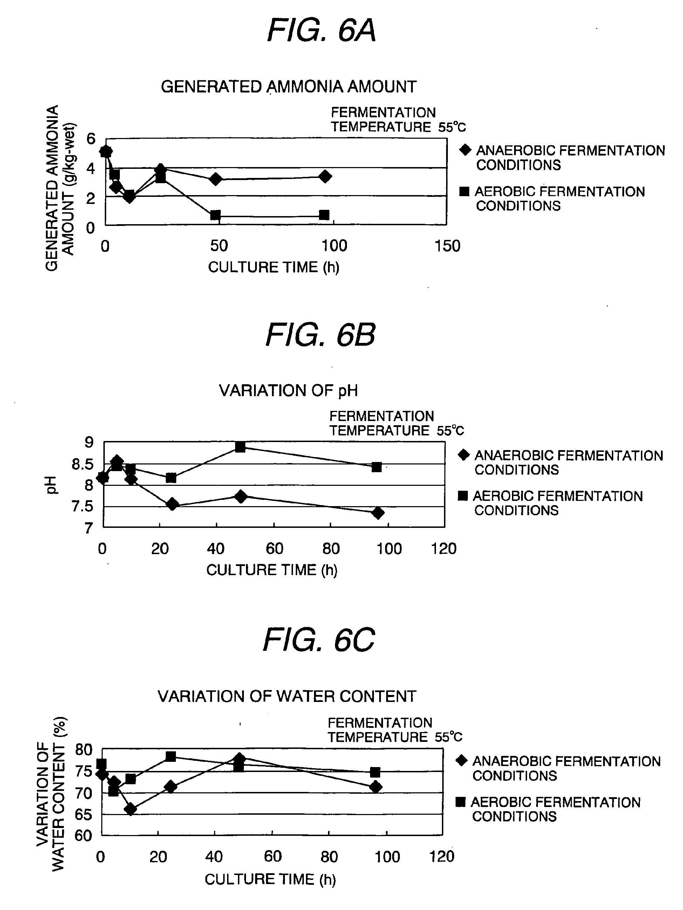

Figs. 6A to 6C show the results of ammonia generation tests carried out under anaerobic fermentation conditions and aerobic fermentation conditions when poultry manure is used as the fermentation material and the bacteria according to the present invention are used. The fermentation temperature is set at 55°C. Figs. 6A, 6B and 6C show the variations of the generated ammonia amount, the pH, and the water content, respectively. The vertical axis of the graph in Fig. 6A shows the generated ammonia amount per one kg of wet poultry manure. Further, the horizontal axis shows the culture time in each of Figs. 6A, 6B and 6C.

An object of the present invention is to remove ammonia by applying a pretreatment to the ammonia fermentation to the extent of preventing ammonia from hindering methane bacteria in the methane fermentation. Further, it is desirable to generate ammonia as much as possible for as short a time as possible in the ammonia fermentation from the viewpoint of the processing speed of the fermentation material and the methanation of organic substances.

From Fig. 6A, it is understood that it is possible to keep the produced ammonia amount higher for a long period of time under the anaerobic fermentation condition in comparison with the aerobic fermentation condition. It shows that the anaerobic fermentation has advantages in the removal of ammonia.

In contrast, from Fig. 6B, the pH tends to shift from an alkaline region to a neutral region in the case of the anaerobic fermentation when the fermentation time is long. It is preferable that a fermentation material is neutral in the methane fermentation and thus the results shown in the figure are desirable ones.

Further, from Fig. 6C, it is understood that the water content does not change from the initial state as far as the fermentation time is about four days (96 hours) under any of the fermentation conditions.

From those results, anaerobic fermentation is suitable for the ammonia fermentation. It is understood that a preferable fermentation time is 6 days or less from the viewpoint of the pH and in particular about 4 days from the viewpoint of the water content.

### [Second Embodiment]

Fig. 2 is a view showing a schematic configuration of a flammable gas production system according to the present invention. The figure shows a case of using a produced flammable gas as the fuel in a combustion apparatus. Although a so-called methane fermenter that produces methane as a representative flammable gas is exemplified here, the present invention is not limited to the methane fermentation. The methane gas produced here contains nonflammable gases, such as carbon dioxide gas, that are discharged by microorganisms during fermentation.

The present system comprises an ammonia fermenter 2 into which a fermentation material 1 is charged first, a methane fermenter 4 into which the fermentation material 1 is charged after the nitrogen component is removed in the ammonia fermenter 2, a heating means 5 for heating the fermentation material 1 in the ammonia fermenter 2 and the methane fermenter 4, a solid-liquid separator 7 to separate fermentation residues 6 discharged from the methane fermenter 4 after the methane fermentation into a digestive juice 8 and a solid component 9, a digestive juice purifying means 10 for purifying the digestive juice 8, a solid component drying means 12 for drying the solid component 9, a carbonizing furnace 13 to carbonize the dried solid component, a combustion apparatus 15 to combust the carbonized gas produced during the carbonizing process, a heat recovery boiler 16 to recover heat of a combustion gas, and a methane gas storage tank 17 to store fermented methane gas. Here, anaerobic fermentation is applied in both the ammonia fermenter and the methane fermenter in the present embodiment. Further, an ammonia fermenter having the structure shown in Fig. 1 is used as the ammonia fermenter 2. Furthermore, the methane fermenter 4 is a dry type fermenter.

Then, the system has a combustion apparatus 18 and an exhaust heat recovery apparatus 19 as a means for using the produced methane gas, and the exhaust gas is discharged through an exhaust stack 20.

The ammonia fermenter 2 and the methane fermenter 4 have temperature control means for controlling the temperatures of the fermentation material 1. Here, the temperature control means includes the heating means 5 for heating the fermentation material 1.

Here, the combustion apparatuses 15 and 18 are energy converters in the broad sense of the term and the thermal energy generated there can be used as heat sources of the temperature control means in the ammonia fermenter 2 and the methane fermenter 4, respectively. In the present embodiment, steam is generated in the heat recovery boiler 16 with the combustion gas supplied from the combustion apparatus 15 and used as the heat source of the heating means 5.

An exhaust port 3 is installed at the upper part of the ammonia fermenter 2 and the generated ammonia gas is discharged. It is also possible to connect the exhaust port 3 to the combustion apparatus 15 or 18, to introduce ammonia gas into the combustion apparatus 15 or 18, and to incinerate and decompose the ammonia gas. By so doing, it is possible to prevent bad odor from being generated.

Further, it is also possible to recover ammonia gas generated in the ammonia fermenter 2 as a hydrogen source and use the ammonia gas as an energy source in a hydrogen energy system.

Here, in the cases of some fermentation materials 1, a hazardous material may be contained in methane gas produced by fermentation and may damage apparatuses. On such occasions, sometimes a means for refining the methane gas produced by fermentation and removing the hazardous material may be installed.

A digestive juice purifying means 10 comprises a water tunnel. Batch-type activated sludge aeration treatment is applied here. The digestive juice purifying means 10 is aimed at purifying the digestive juice 8 after the methane fermentation treatment by simple operations at a low cost, is provided with two aeration means 51 and processes the digestive juice batch-wise once a day. A sand filter bed 11 to remove residues of the digestive juice 8 and a dilution tank (not shown in the figure) to dilute wastewater may be attached to the digestive juice purifying means 10.

A conventional sewage treatment tank has a complicated structure because sewage contains a nitrogen component and the complexity of the structure has caused both the initial and the running costs to increase. In contrast, the present system facilitates the purification of the digestive juice 8 and makes it possible to purify the digestive juice 8 with only one digestive juice purifying means 10 since the nitrogen component contained in a fermentation material 1 is removed in the ammonia fermenter 2 beforehand and the fermentation residues 6 discharged from the methane fermenter 4 are separated into the digestive juice 8 and the solid component 9.

The water content of the solid component 9 produced after the fermentation residues 6 discharged from the dry type methane fermenter 4 is subjected to solid-liquid separation is about 50% to 60%. The solid component drying means 12 dries the solid component 9 of a high water content in the atmosphere of 300°C or higher and facilitates the succeeding carbonization. The water vapor 55 generated from the solid component 9 is discharged outside the solid component drying means 12. Further, dust generated from the solid component 9 is recovered with a cyclone 56.

With the present system, it is not necessary to excessively lower the water content in the solid component 9 produced by the solid-liquid separation of the fermentation residues 6 and the charged energy required by the solid-liquid separator 7 can be reduced. The energy recovered here is used for heating the fermentation material in the dry type methane fermenter 4.

The carbonizing furnace 13 is of an internal self-heating type. In the carbonizing furnace 13, the atmospheric temperature is 600 to 900°C and the solid component 9 is carbonized after dried by the solid component drying means 12. Among the carbonized gases (CO, H₂O, CH₄, etc.) generated here and products 52 containing carbide, the carbonized gases burn in the combustion apparatus 15. The heat generated at the time is recovered with the heat recovery boiler 16 and used as the heat source of the heating means 5. The heat of the heating means 5 can be used for heating in the ammonia fermenter 2 and the methane fermenter 4. A part 53 of the carbonized gases generated in the carbonizing furnace 13 flows back to the solid component drying means 12 and is used as the heat source.

As stated above, with the carbonizing furnace 13 and the combustion apparatus 15, it is possible to recover energy also from the solid component 9 derived from fermentation residues 6 and to reduce the volume of the solid component 9 to 20 to 30%.

An ash component 14 is the final product generated from the present system, which can be used as a valuable resource such as a snow-melting agent and is very easy to handle.

An exhaust gas 54 from which heat is recovered with the heat recovery boiler 16 is discharged from the exhaust stack 20.

The fermentation residues 6 discharged from the methane fermenter 4 are separated into the digestive juice 8 and the solid component 9, and the digestive juice 8 is purified by the batch-type activated sludge aeration method in the digestive juice purifying means 10.

Whether or not the digestive juice 8 is suitable for biological treatment is judged by a biochemical oxygen demand (BOD), a chemical oxygen demand (COD), or a total organic carbon (TOC) and, when the concentration of the BOD is high, the digestive juice 8 is judged to be suitable for the biological treatment.

With the present system, since a nitrogen component is removed in the ammonia fermenter 2, it is possible to almost satisfy the regulation on drainage discharge based on the Water Pollution Prevention Law by biological treatment. A case of requiring a coagulant is only exceptional.

It is possible to reduce the water treatment cost that has been a large amount of money in the case of a conventional technology by removing a nitrogen component with the ammonia fermenter 2 according to the present invention.

Not only in the case of a biomass methane fermentation but also in the case where a material having a low energy quality level is converted into a high-quality energy material, the price of the converted material per unit energy tends to increase. For that reason, there is a need to construct a system to recover energy as much as possible from the fermentation material 1 as a biomass. The present system makes it possible to recover energy from the solid component 9 derived from the fermentation residues 6 and also to reduce the volume.

As the fermentation material 1, food waste or livestock excreta containing an organic nitrogen component abundantly may also be used. The present invention is very effective also for, among the livestock excreta in particular, poultry manure that has heretofore been regarded as hardly fermented.

A technological feature of the present system is that it is possible to decompose various organic substances efficiently and to produce a flammable gas stably since the organic nitrogen component can be removed beforehand in an ammonia fermenter according to the present invention at a low cost regardless of the amount of the nitrogen component included in the fermentation material 1.

The fermentation residues 6 extracted from the methane fermenter 4 are separated into a digestive juice 8 and a solid component 9. The digestive juice 8 is discharged in the manner of satisfying the regulation stipulated by the Water Pollution Prevention Law after subjected to biological treatment in a batch-type activated sludge aeration tank of the digestive juice purifying means 10. The solid component 9 is subjected to additional recovery of energy and volume reduction with the carbonizing furnace 13 and the remaining carbide can be used as a valuable resource such as a snow-melting agent.

In the present embodiment shown in Fig. 2, the ammonia fermenter 2 is an anaerobic fermenter and a vertical type. In the figure, the ammonia fermenter 2 is divided into three stages of an upper stage, a middle stage, and a lower stage. The fermentation material 1 is dropped to respective stages at prescribed intervals and is mixed. The fermentation material 1 in the ammonia fermenter 2 is heated by an external heat source, for example, heat obtained from a carbonized gas combustion apparatus 15 and the fermentation temperature is controlled to a constant temperature.

The fermentation material 1 is retained in the ammonia fermenter 2 for four days and thereby 70% to 80% of the nitrogen component in the fermentation material 1 is converted into ammonia and denitrified. Microorganism groups involved in the ammonia fermentation are analyzed and the structure of the ammonia fermenter 2 is studied in order to make the ammonia fermentation efficient. As a result, an ammonia yield of 85% and a carbon class reduction ratio of 15% can be obtained when the retention time of the fermentation material 1 is four days.

Methane fermentation is applied after the ammonia fermentation is applied under the conditions, and resultantly a fermented methane gas of 80 to 100 m³ per one ton of the fermentation material is obtained. As a result of ammonia removal in the ammonia fermenter 2, the ammonia concentration in the methane fermenter 4 of the succeeding step is 0.35% or less and ammonia can be prevented from hindering methane bacteria.

The methane fermenter 4 is an anaerobic dry type. A paddle type agitator is incorporated into the methane fermenter 4. At all times, a part of the fermentation material 1 is transferred from the methane fermenter 4 to the heating means 5, heat exchange is carried out, and the temperature in the methane fermenter 4 is controlled to a constant temperature.

The fermentation material 1 in the methane fermenter 4 is analyzed at prescribed intervals, the charging rate and the charging amount of the fermentation material 1 supplied to the methane fermenter 4 are controlled so that the fermentation material 1 in the methane fermenter 4 may be under desirable conditions, namely so that the pH may be around 8 and the ammonia concentration may be 3500 mg/kg or less, and methane gas is produced efficiently. By so doing, a very high-energy conversion efficiency of 75% or more is realized on the basis of an enthalpy charged to the methane fermenter 4.

The energy conversion efficiency here is the ratio of the sum of the enthalpy of the flammable gas obtained in the methane fermentation and the enthalpy obtained by carbonizing the residues to the total enthalpy of the fermentation material 1.

An anaerobic ammonia fermenter according to the present invention is described hereunder.

In Fig. 2, the fermentation material 1 is sent to the anaerobic ammonia fermenter 2 and retained in the ammonia fermenter 2 for several days. During this period, the fermentation material 1 is heated to a prescribed temperature by heat supplied from the heating means 5 or the like in order to obtain a temperature environment most suitable for the ammonia fermentation, and the ammonia fermentation is accelerated.

A means for conveying the fermentation material 1 from the ammonia fermenter 2 to the methane fermenter 4 or exterior is called a fermentation material conveying means.

With the system according to the present invention, unlike with a wet type fermentation system using dilution water, the discharged amount of a digestive juice 8 reduces considerably, the capacity of the digestive juice purifying means 10 prepared thereafter also reduces considerably, and it is estimated that the economic effect is very large and the operation is simple in commercial use and tends to be prevalent.

### [Third Embodiment]

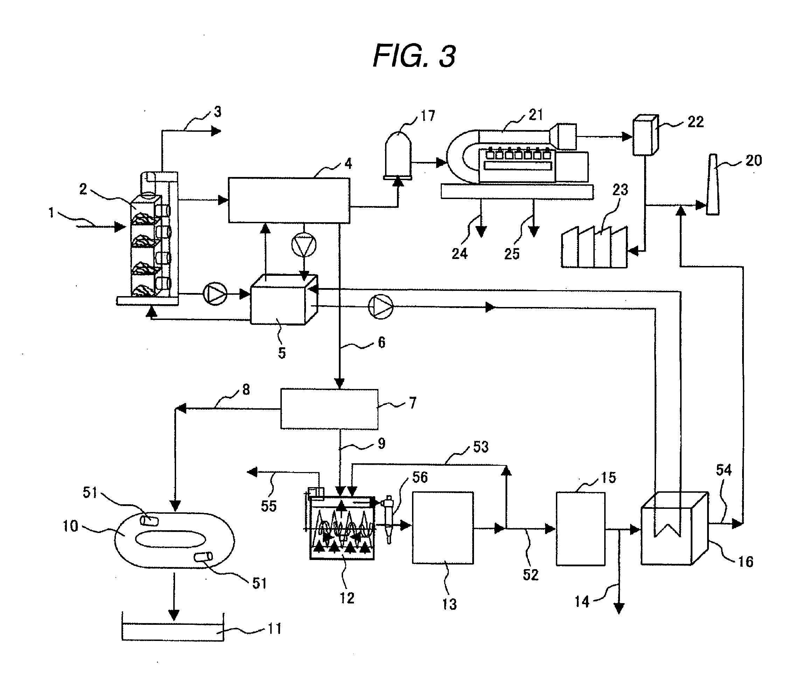

Fig. 3 shows another example of an energy converter used in a flammable gas production system according to the present invention. The difference from the energy converter used in the embodiment shown in Fig. 2 is that the methane gas produced from a methane fermenter 4 is used as a fuel for a gas engine 21. On this occasion, mechanical energy can be obtained with the gas engine 21. Then, electricity can be generated by using the mechanical energy and electric energy 25 can be obtained. Further, thermal energy 24 can also be obtained by the combustion of methane gas. A device that can obtain mechanical energy, electric energy and thermal energy from methane gas as stated above is called an energy converter.

It is also possible to use electric energy or thermal energy generated with the energy converter as the heat source for the temperature control means of the ammonia fermenter 2 and the methane fermenter 4.

Further, it is also possible to install an exhaust heat recovery device 22 for recovering heat from the exhaust gas of the gas engine 21. It is also possible to use the exhaust heat for heating the ammonia fermenter 2 and the methane fermenter 4. Furthermore, it is also possible to use the exhaust gas of the gas engine 21 as the heat source of a greenhouse 23 or a carbon dioxide source.

Here, although the energy converter using the gas engine 21 is explained in the present embodiment, the similar energy conversion can be carried out even when a fuel cell is used instead of the gas engine 21.

### [Array Table Free Text]

### Arrangement number 1: Base arrangement (1457bp) of 16s rDNA of Tepidimicrobium sp. strain HUT8118:

GACGAACGCTGGCGGCGTGCCTAACACATGCAAGTCGAGCGAGGGAT
TTATGACGGATTCCTTCGGGATGAAGACATAAATCACCTAGCGGCGGA
CGGGTGAGTAACGCGTGAGGAACCTACCTCCTACAAGGGGATAGCCT
CGGGAAACTGGGATTAATACCCTATGACACTTAAGGATCGCATGATCTT
TAGGTTAAAGCGATATAGCGGTAGGAGATGGCCTCGCGTCCCATTAGC
TAGTTGGTGAGGTAACGGCTTACCAAGGCGACGATGGGTAGCCGGCC
TGAGAGGGTGAACGGCCACACTGGAACTGAGACACGGTCCAGACTCC
TACGGGAGGCAGCAGTGGGGAATATTGCACAATGGGCGAAAGCCTGA
TGCAGCGACGCCGCGTGAGCGAAGAAGGCCTTCGGGTCGTAAAGCTC
TGTCCTATGGGACGATAATGACGGTACCATAGGAGGAAGCCCCGGCT
AACTACGTGCCAGCAGCCGCGGTAATACGTAGGGGGCGAGCGTTGTC

CGGAATTACTGGGCGTAAAGGGTACGCAGGCGGCTAGGTAAGTCAGA
TGTGAAATCCATGGGCTCAACCCATGTAAGCATTTGAAACTGCTTAGCT
TGAGTTAAGGAGAGGAAAGTGGAATTCCTGGTGTAGCGGTGAAATGCG
TAGATATCAGGAGGAATACCAGTGGCGAAGGCGACTTTCTGGCCTTAA
ACTGACGCTGAGGTACGAAAGCGTGGGGAGCAAACAGGATTAGATAC
CCTGGTAGTCCACGCCGTAAACGATGAGTGCTAGGTGTCGGGGGTTT
AACCTTCGGTGCCGCAGTTAACACATTAAGCACTCCGCCTGGGGAGTA
CGTTCGCAAGAATGAAACTCAAAGGAATTGACGGGGACCCGCACAAG
CAGCGGAGCATGTGGTTTAATTCGATGCAACGCGAAGAACCTTACCAG
GGCTTGACACCCCTCTGCCGGGACTAGAGATAGTCCTTTCCCTTCGGG
GACGGAGGAGACAGGTGGTGCATGGTTGTCGTCAGCTCGTGTCGTGA
GATGTTGGGTTAAGTCCCGCAACGAGCGCAACCCTTGCCTTTAGTTGC
CAGCACTTTAGGTGGGCACTCTAGAGGGACTGCCGATGACAAATCGG
AGGAAGGTGGGGATGACGTCGAATCGTCATGCCCTTTATGTCCTGGG
CTACACACGTGCTACAATGGTCGGTACAACGGGTAGCAAGCGAGCGA
TCGTGAGCCAATCCCTAAAAGCCGATCTCAGTTCGGATTGCAGGCTGC
AACTCGCCTGCATGAAGTCGGAGTTGCTAGTAATCGCGGATCAGCATG
CCGCGGTGAATGCGTTCCCGGGTCTTGTACACACCGCCCGTCACACC
ATGGGAGTTAGCAATACCCGAAGCCAGTGAGCTAACCTTAAAGGGGG
CAGCTGTCGAAGGTAGGGCTAATGACTGGGGTG

### Arrangement number 2: Base arrangement (1460bp) of 16s rDNA of Tepidimicrobium sp. strain HUT8119:

GACGAACGCTGGCGGCGTGCCTAACACATGCAAGTCGAGCGAAGTGC
TTTAAGTGGAATCCTTCGGGAGGAAACTTGAAGCGCTTAGCGGCGGAC
GGGTGAGTAATACGTGAGGAACCTACCTTCCACAAGGGGATAGCCTC
GGGAAACCGGGATTAATACCCTATGACACCTAAGAGTCGCATGATACC
TAGGTTAAAGCGAATTAGCGGTGGAGATGGCCTCACGTCCCATTAGC
TAGTTGGTGAGGTAACGGCTCACCAAGGCGACGATGGGTAGCCGGCC
TGAGAGGGTGACCGGCCACACTGGGACTGAGACACGGCCCAGACTCC
TACGGGAGGCAGCAGTGGGGAATATTGCACAATGGGCGAAAGCCTGA
TGCAGCGACGCCGCGTGAGCGAAGAAGGCCTTCGGGTCGTAAAGCTC
TGTCCTATGGGAAGATAATGACGGTACCATAGGAGGAAGCCCCGGCTA
ACTACGTGCCAGCAGCCGCGGTAATACGTAGGGGGCGAGCGTTGTCC

GGAATGACTGGGCGTAAAGGGTACGTAGGCGGCTGAGTAAGTCAGAT
GTGAAAGTCTAGGGCTCAACCCTAGTGAGCATTTGAAACTGCTTAGCTT
GAGTCGAGGAGAGGAAGGTGGAATTCCTGGTGTAGCGGTGAAATGCG
TAGATATCAGGAGGAATACCAGTGGCGAAGGCGACCTTCTGGCCTTG
GACTGACGCTGAGGTACGAAAGCGTGGGGAGCAAACAGGATTAGATA
CCCTGGTAGTCCACGCCGTAAACGATGAGTGCTAGGTGTCGGGGGAG
ATATCCTTCGGTGCCGAAGTTAACACATTAAGCACTCCGCCTGGGGAG
TACGGTCGCAAGACTGAAACTCAAAGGAATTGACGGGGACCCGCACA
AGCAGCGGAGCATGTGGTTTAATTCGATGCAACGCGAAGAGCCTTACC
AGGGCTTGACATCCCTCTGACCGGTGTAGAGATACACCCTTCTTCCTT
CGGGAAGACAGAGGAGACAGGTGGTGCATGGTTGTCGTCAGCTCGTG
TCGTGAGATGTTGGGTTAAGTCCCGCAACGAGCGCAACCCTTGCCTCT
AGTTGCCAGCACTTTGGGTGGGCACTCTAGAGGGACTGCCGGCAAAA
AGTCGGAGGAAGGTGGGGATGACGTCAAATCATCATGCCCTTTATGTC
CTGGGCTACACACGTGCTACAATGGTCGGTACAAAGGGCAGCGACCT
AGCGATAGGAAGCGAATCCCAAAAAGCCGATCTCAGTTCGGATTGCAG
GCTGCAACTCGCCTGCATGAAGCCGGAGTTGCTAGTAATCGCGGATCA
GCATGCCGCGGTGAATACGTTCCCGGGTCTTGTACACACCGCCCGTC
ACACCATGGGAGTCAGCAATGCCTGAAGCCAGTGAGCTAACCGTAAG
GGGGCAGCTGTCGAAGGCAGGGCTGATGACTGGGGTG

[Array Table]
Array table

## Claims

1. An ammonia fermenter for generating ammonia gas comprising:
a container for charging thereinto a fermentation material including at least one kind of bacteria selected from among bacteria named *Tepidimicrobium* sp. strain HUT8118 and deposited under a reference number NITE AP-390 at Patent Microorganisms Depositary in Incorporated Administrative Agency National Institute of Technology and Evaluation, bacteria named *Tepidimicrobium* sp. strain HUT8119 and deposited under a reference number NITE AP-391 at Patent Microorganisms Depositary in Incorporated Administrative Agency National Institute of Technology and Evaluation, *Tepidimicrobium ferriphilum, Clostridium ultunense,* Peptostreptococcaceae bacterium 19gly, Clostridium sp. PO, *Sporanaerobacter acetigenes* Lup33, and *Acetanaerobacter* sp.;
a temperature control means for controlling a temperature of said fermentation material, and
an exhaust port for discharging ammonia gas generated in said container outside said container.

2. The ammonia fermenter according to Claim 1, which is an anaerobic fermenter.

3. The ammonia fermenter according to Claim 1 or 2, which comprises a mixing means for mixing said fermentation material, wherein said mixing means includes at least one of a free fall means and a mechanical agitation means.

4. An apparatus for producing a flammable gas comprising:
the ammonia fermenter according to any one of Claims 1 to 3;
a flammable gas fermenter for further fermenting said fermentation material denitrified in the ammonia fermenter to extract a flammable gas, and
a means for transferring the fermentation material from the ammonia fermenter to the flammable gas fermenter.

5. The apparatus according to Claim 4, which includes an energy converter for generating electric energy or thermal energy from said flammable gas, wherein said temperature control means in said ammonia fermenter uses said electric energy or said thermal energy supplied from said energy converter.

6. The apparatus according to Claim 5, wherein an odorous component generated from said ammonia fermenter is taken into said energy converter and removed at said energy converter.

7. A system for producing a flammable gas comprising:
the apparatus according to any one of Claims 4 to 6;
a solid-liquid separating means for separating fermentation residues discharged from said flammable gas fermenter into a digestive juice and a solid component;
a digestive juice purifying means for purifying said digestive juice, and
a solid component drying means for drying said solid component.

8. The system according to Claim 7, which includes:
a carbonizing furnace for carbonizing said solid component dried by said solid component drying means and
a combustion apparatus for combusting the carbonized gas generated from the carbonizing furnace.

9. A method for operating the apparatus according to any one of Claims 4 to 6, which comprises steps for controlling of:
finishing retention of said fermentation material in said ammonia fermenter and transferring said fermentation material into said flammable gas fermenter when the ammonia concentration of said fermentation material in said ammonia fermenter comes to 3500 mg/kg or lower.
